(19) Europäisches Patentamt
European Patent Office
Office européen des brevets

(11) Veröffentlichungsnummer: **0 327 977**
**A1**

# (12) EUROPÄISCHE PATENTANMELDUNG

(21) Anmeldenummer: **89101842.6**

(22) Anmeldetag: **03.02.89**

(51) Int. Cl.4: **C07D 401/12 , C07D 405/12 , C07D 409/12 , C07D 207/34 , A01N 43/36 , A01N 43/40**

(30) Priorität: **11.02.88 DE 3804127**

(43) Veröffentlichungstag der Anmeldung: **16.08.89 Patentblatt 89/33**

(84) Benannte Vertragsstaaten: **BE CH DE ES FR GB IT LI NL**

(71) Anmelder: **BAYER AG**

**D-5090 Leverkusen 1 Bayerwerk(DE)**

(72) Erfinder: **Wollweber, Detlef, Dr.**
**Paul-Ehrlich-Strasse 17**
**D-5600 Wuppertal 1(DE)**
Erfinder: **Brandes, Wilhelm, Dr.**
**Eichendorffstrasse 3**
**D-5653 Leichlingen(DE)**

(54) **1-Aminomethyl-3-(2,4-difluor-3-chlorphenyl)-4-cyanopyrrole.**

(57) 1-Aminomethyl-3-(2,4-difluor-3-chlorphenyl)-4-cyanopyrrole der allgemeinen Formel (I),

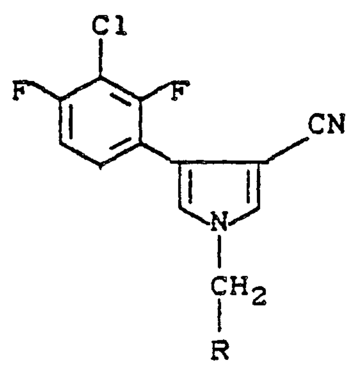

(I)

in welcher

R die in der Beschreibung gegebene Bedeutung hat und ihre Verwendung in Pflanzenschutzmitteln.

Die neuen 1-Aminomethyl-3-(2,4-difluor-3-chlorphenyl)-4-cyano-pyrrole sind durch die Formel (I) definiert. Sie sind nach Analogieverfahren herstellbar, z.B. aus 3-(2,4-Difluor-3-chlorphenyl)-4-cyano-pyrrol und Formaldehyd und geeigneten Aminen oder aus geeigneten 3-(2,4-Difluor-3-chlorphenyl)-4-cyano-pyrrolen, die noch neu sind, mit geeigneten Aminen. Die ebenfalls noch neuen Ausgangspyrrole erhält man z.B. aus 3-(2,4-Difluor-3-chlorphenyl)-4-cyano-pyrrol und Formaldehyd gegebenenfalls in Gegenwart eines Verdünnungsmittels, gegebenenfalls in Gegenwart eines Halogenierungsmittels und gegebenenfalls in Gegenwart eines Säurebindemittels.

EP 0 327 977 A1

## 1-Aminomethyl-3-(2,4-difluor-3-chlorphenyl)-4-cyano-pyrrole

Die Erfindung betrifft neue 1-Aminomethyl-3-(2,4-difluor-3-chlorphenyl)-4-cyano-pyrrole, mehrere Verfahren zu ihrer Herstellung sowie ihre Verwendung als Schädlingsbekämpfungsmittel, insbesondere als Fungizide, und neue Zwischenprodukte.

Es ist bekannt, daß bestimmte 3-Aryl-pyrrole, wie beispielsweise das 4-Cyano-3-(2,3-dichlorphenyl)-pyrrol gute fungizide Eigenschaften besitzen (vgl. z.B. EP 174 910 oder EP 182 738 oder EP 133 247).

Außerdem sind 1-Aminomethyl-3-aryl-4-cyano-pyrrole mit fungizider Wirkung aus vorgängigen, aber nicht vorveröffentlichten deutschen Anmeldungen P 3 702 852.9 und P 3 702 853.7 bekannt.

Es wurden neue 1-Aminomethyl-3-(2,4-difluor-3-chlorphenyl)-4-cyano-pyrrole der allgemeinen Formel (I),

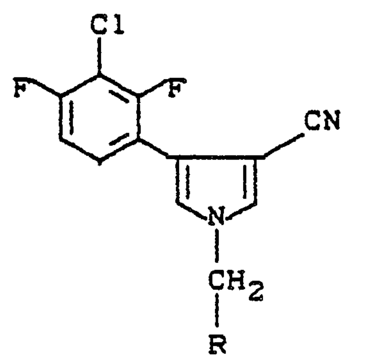

(I)

in welcher
R für einen Rest

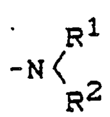 ; 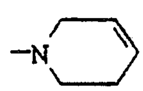 ; 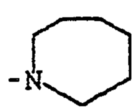

oder

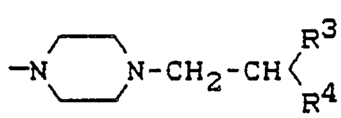

steht,
wobei
$R^1$ für gegebenenfalls substituiertes Alkyl, für Alkenyl, Alkinyl oder Cycloalkyl oder für jeweils gegebenenfalls substituiertes Aralkyl oder Aryl steht,
$R^2$ für jeweils gegebenenfalls substituiertes Heterocyclylalkyl, Heterocyclylalkenyl, Heterocyclylalkinyl oder Heterocyclyl, für Dialkylaminoalkyl oder für jeweils gegebenenfalls substituiertes Cycloalkylalkyl oder Phenethyl steht,
$R^3$ für Wasserstoff oder Alkyl steht und
$R^4$ für Cyano, Alkanoyl, Alkoxycarbonyl, Alkylaminocarbonyl, Dialkylaminocarbonyl, für Alkylsulfinyl, für Alkylsulfonyl, für Phenylsulfinyl oder für Phenylsulfonyl steht,
gefunden.

Weiterhin wurde gefunden, daß man die neuen 1-Aminomethyl-3-(2,4-difluor-3-chlorphenyl)-4-cyano-pyrrole der allgemeinen Formel (I),

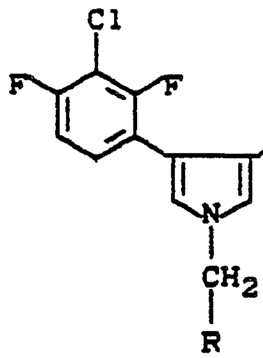

(I)

in welcher
R für einen Rest

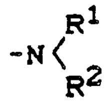 ; 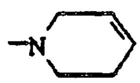 ; 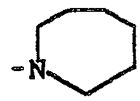

oder

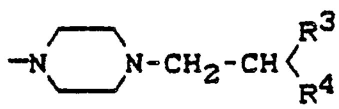

steht,
wobei
$R^1$ für gegebenenfalls substituiertes Alkyl, für Alkenyl, Alkinyl oder Cycloalkyl oder für jeweils gegebenenfalls substituiertes Aralkyl oder Aryl steht,
$R^2$ für jeweils gegebenenfalls substituiertes Heterocyclylalkyl, Heterocyclylalkenyl, Heterocyclylalkinyl oder Heterocyclyl, für Dialkylaminoalkyl oder für jeweils gegebenenfalls substituiertes Cycloalkylalkyl oder Phenethyl steht,
$R^3$ für Wasserstoff oder Alkyl steht und
$R^4$ für Cyano, Alkanoyl, Alkoxycarbonyl, Alkylaminocarbonyl, Dialkylaminocarbonyl, für Alkylsulfinyl, für Alkylsulfonyl, für Phenylsulfinyl oder für Phenylsulfonyl steht,
erhält, wenn man
(a) das 3-(2,4-Difluor-3-chlorphenyl)-4-cyano-pyrrol der Formel (II)

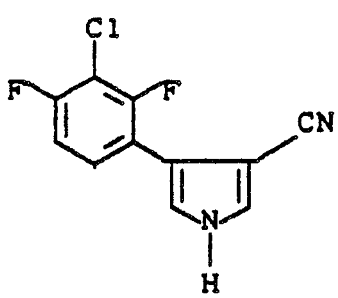

(II)

mit Formaldehyd und Aminen der Formel (III),
R-H (III)
in welcher
R die oben angegebene Bedeutung hat,

gegebenenfalls in Gegenwart eines Verdünnungsmittels und gegebenenfalls in Gegenwart eines Reaktionshilfsmittels umsetzt oder wenn man,

(b) 3-(2,4-Difluor-3-chlorphenyl)-4-cyano-pyrrole der Formel (IV),

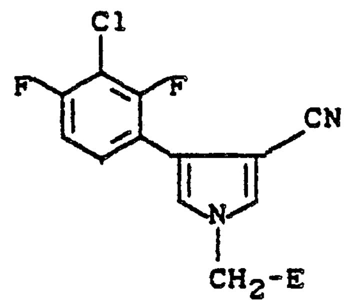

(IV)

in welcher

E für eine elektronenanziehende Abgangsgruppe steht,

mit Aminen der Formel (III),

H-R (III)

in welcher

R die oben angegebene Bedeutung hat,

gegebenenfalls in Gegenwart eines Verdünnungsmittels und gegebenenfalls in Gegenwart eines Säurebindemittels umsetzt.

Schließlich wurde gefunden, daß die neuen 1-Aminomethyl-3-(2,4-difluor-3-chlorphenyl)-4-cyano-pyrrole der allgemeinen Formel (I) gute Wirkung gegen Schädlinge vor allem gegen Pilze besitzen.

Die erfindungsgemäßen 1-Aminomethyl-3-(2,4-difluor-3-chlorphenyl)-4-cyano-pyrrole sind durch die Formel (I) allgemein definiert. Bevorzugt sind Verbindungen der Formel (I)< bei welchen

R für einen Rest

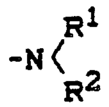 ; 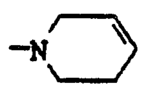 ; 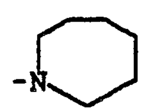

oder

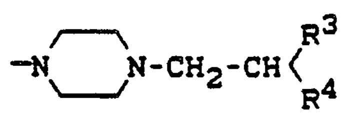

steht,

wobei

$R^1$ für gegebenenfalls substituiertes, geradkettiges oder verzweigtes Alkyl mit 1 bis 6 Kohlenstoffatomen steht, wobei als Substituenten infrage kommen: Cyano, Cycloalkyl mit 3 bis 7 Kohlenstoffatomen, jeweils geradkettiges oder verzweigtes Alkoxy, Alkylcarbonyl, Alkoxycarbonyl, Alkylaminocarbonyl, Dialkylaminocarbonyl, Alkylsulfinyl, Alkylsulfonyl oder Dialkylamino mit jeweils 1 bis 6 Kohlenstoffatomen in den einzelnen Alkylteilen; außerdem für jeweils geradkettiges oder verzweigtes Alkenyl oder Alkinyl mit jeweils 3 bis 6 Kohlenstoffatomen, für Cycloalkyl mit 3 bis 7 Kohlenstoffatomen oder für jeweils gegebenenfalls einfach bis mehrfach, gleich oder verschieden im Phenylteil substituiertes Phenyl oder Benzyl steht, wobei als Substituenten jeweils infrage kommen: Halogen, Cyano, jeweils geradkettiges oder verzweigtes Alkyl, Alkoxy, Halogenalkyl, Halogenalkoxy, Alkylcarbonyl oder Alkoxycarbonyl mit jeweils 1 bis 6 Kohlenstoffatomen in den einzelnen Alkylteilen und im Fall des Halogenalkyl- oder Halogenalkoxy-Restes mit jeweils 1 bis 9 gleichen oder verschiedenen Halogenatomen,

$R^2$ für jeweils geradkettiges oder verzweigtes Heterocyclylalkyl mit 1 bis 6 Kohlenstoffatomen im Alkylteil,

Heterocyclylalkenyl mit 3 bis 6 Kohlenstoffatomen im Alkenylteil, Heterocyclylalkinyl mit 3 bis 6 Kohlenstoffatomen im Alkinylteil oder Heterocyclyl steht, wobei Heterocyclyl jeweils für einen 5- bis 7-gliedrigen, gegebenenfalls einfach bis mehrfach, gleich oder verschieden substituierten, ungesättigen oder gesättigten Heterocyclus mit 1 bis 3 Heteroatomen, insbesondere Stickstoff, Sauerstoff oder Schwefel steht und wobei als Substituenten jeweils infrage kommen: Halogen, Cyano, jeweils geradkettiges oder verzweigtes Alkyl, Alkoxy, Halogenalkyl, Halogenalkoxy, Alkylcarbonyl oder Alkoxycarbonyl mit jeweils 1 bis 6 Kohlenstoffatomen in den einzelnen Alkylteilen und im Fall des Halogenalkyl- oder Halogenalkoxy-Restes mit jeweils 1 bis 9 gleichen oder verschiedenen Halogenatomen; außerdem für geradkettiges oder verzweigtes Dialkylaminoalkyl mit jeweils 1 bis 6 Kohlenstoffatomen in den einzelnen Alkylteilen steht, ferner für im Cycloalkylteil gegebenenfalls einfach bis mehrfach, gleich oder verschieden durch Halogen und/oder geradkettiges oder verzweigtes Alkyl mit 1 bis 4 Kohlenstoffatomen substituiertes Cycloalkylalkyl mit 1 bis 6 Kohlenstoffatomen im geradkettigen oder verzweigten Alkylteil und 3 bis 7 Kohlenstoffatomen im Cycloalkylteil oder für im Phenylteil gegebenenfalls einfach bis mehrfach, gleich oder verschieden durch Cyano, Halogen und/oder jeweils geradkettiges oder verzweigtes Alkyl oder Alkoxy mit jeweils 1 bis 4 Kohlenstoffatomen substituiertes Phenylethyl steht,

$R^3$ für Wasserstoff oder für geradkettiges oder verzweigtes Alkyl mit 1 bis 6 Kohlenstoffatomen steht und

$R^4$ für Cyano oder für jeweils geradkettiges oder verzweigtes Alkanoyl, Alkoxycarbonyl, Alkylaminocarbonyl, Dialkylaminocarbonyl, Alkylsulfinyl oder Alkylsulfonyl mit jeweils 1 bis 6 Kohlenstoffatomen in den einzelnen Alkylteilen oder für Phenylsulfinyl oder Phenylsulfonyl steht.

Besonders bevorzugt sind Verbindungen der Formel (I), bei welchen

R für einen Rest

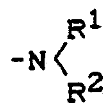 ; 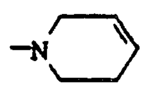 ; 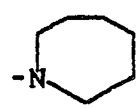

oder

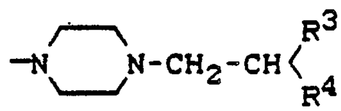

steht,

wobei

$R^1$ für jeweils gegebenenfalls substituiertes Methyl, Ethyl, n- oder i-Propyl sowie n-, i- oder s-Butyl steht, wobei als Substituenten jeweils infrage kommen: Cyano, Methoxy, Ethoxy, Methylcarbonyl, Ethylcarbonyl, Methoxycarbonyl, Ethoxycarbonyl, Methylaminocarbonyl, Dimethylaminocarbonyl, Ethylaminocarbonyl, Diethylaminocarbonyl, Methylsulfinyl, Methylsulfonyl, Dimethylamino, Diethylamino oder Dipropylamino, Dibutylamino oder Cyclohexyl; außerdem für Allyl, n- oder i-Butenyl, Propargyl, n- oder i-Butinyl, für Cyclopentyl, Cyclohexyl, Cyclopropyl oder für jeweils gegebenenfalls ein- bis dreifach, gleich oder verschieden im Phenylteil substituiertes Benzyl oder Phenyl steht, wobei als Substituenten infrage kommen: Fluor, Chlor, Brom, Cyano, Methyl, Ethyl, n- oder i-Propyl, n-, i- oder s-Butyl, Methoxy, Ethoxy, Trifluormethyl, Trifluormethoxy, Acetyl, Methoxycarbonyl oder Ethoxycarbonyl,

$R^2$ für jeweils gegebenenfalls im Heterocyclylteil ein- oder zweifach, gleich oder verschieden substituiertes Heterocyclylmethyl, Heterocyclylethyl oder Heterocyclylpropyl steht, wobei als Heterocyclylrest jeweils einer der folgenden Reste infrage kommt

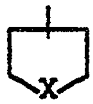 ; 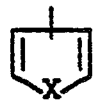 ; 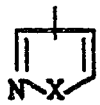 ; 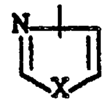 ; 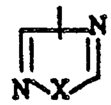 ;

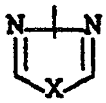 ; 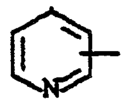 ; 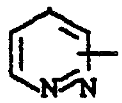 ; 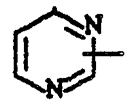 ; 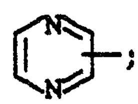 ;

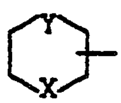 und 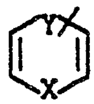 ,

wobei X jeweils für Sauerstoff, Schwefel oder eine NH-Gruppe steht und Y jeweils für Sauerstoff, Schwefel, eine NH-Gruppe oder eine $CH_2$-Gruppe steht und wobei als Substituenten jeweils infrage kommen: Fluor, Chlor, Brom, Cyano, Methyl, Ethyl, n- oder i-Propyl, n-, i-, s- oder t-Butyl, Methoxy, Ethoxy, Trifluormethyl, Trifluormethoxy, Acetyl, Methoxycarbonyl oder Ethoxycarbonyl; außerdem für Dimethylaminoethyl, Diethylaminoethyl, Dimethylaminopropyl, Diethylaminopropyl, Dipropylaminopropyl, Dibutylaminopropyl, Dibutylaminobutyl, Dibutylaminoethyl, Dipropyl aminoethyl, Dimethylaminobutyl, Diethylamino butyl, Dimethylaminopentyl, Diethylaminopentyl, für jeweils gegebenenfalls ein- bis dreifach, gleich oder verschieden durch Methyl, Ethyl und/oder Isopropyl substituiertes Cyclohexylmethyl oder Cyclohexylethyl oder für gegebenenfalls ein- bis dreifach, gleich oder verschieden durch Fluor, Chlor, Brom, Cyano, Methyl, Ethyl, n- oder i-Propyl, n-, i-, s- oder t-Butyl, Methoxy, Ethoxy, n- oder i-Propoxy und n-, i-, s- oder t-Butoxy substituiertes Phenethyl steht,

$R^3$ für Wasserstoff, Methyl, Ethyl, n- oder i-Propyl sowie n-, i-, s- oder t-Butyl steht und

$R^4$ für Cyano, Acetyl, Propionyl, Methoxycarbonyl, Ethoxycarbonyl, Methylaminocarbonyl, Dimethylaminocarbonyl, Ethylaminocarbonyl, Diethylaminocarbonyl, Dipropylaminocarbonyl, Dibutylaminocarbonyl, Methylsulfinyl, Ethylsulfinyl, Methylsulfonyl, Ethylsulfonyl, Phenylsulfinyl oder Phenylsulfonyl steht.

Ganz besonders bevorzugt sind Verbindungen der Formel (I), bei welchen

R für einen Rest

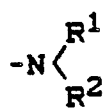 ; 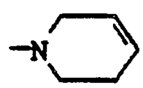 ; 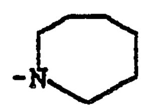

oder

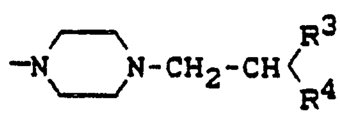

steht,

wobei

$R^1$ für Methyl, Ethyl, n- oder i-Propyl, n-, i-oder s-Butyl, Allyl, Propargyl, für Cyclohexyl, Cyclopropyl, Cyclopentyl, Cyclohexylmethyl, Phenyl, Benzyl oder für Cyanethyl steht,

$R^2$ für jeweils gegebenenfalls ein- bis dreifach, gleich oder verschieden durch Chlor und Methyl substituiertes Heterocyclylmethyl, Heterocyclylethyl oder Heterocyclylpropyl steht, wobei als Heterocyclylreste infrage kommen:

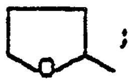 ; 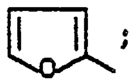 ; 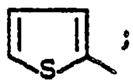 ; 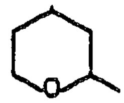 ; 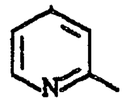 ;

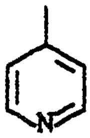 oder 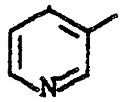 ;

oder für jeweils gegebenenfalls ein- bis dreifach, gleich oder verschieden durch Methyl und Ethyl substituiertes 2-Heterocyclylethyl, 2-Heterocyclylpropyl oder 3-Heterocyclylpropyl steht, wobei als Heterocyclylreste infrage kommen:

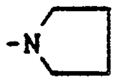 ; 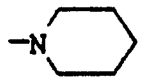 ; 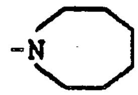 ; 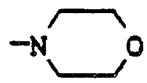 ;

außerdem für Diethylaminoethyl, Dimethylaminopropyl, Diethylaminopropyl, Dibutylaminopropyl oder Diethylaminopentyl, für Cyclohexylmethyl oder für Phenethyl steht,
$R^3$ für Wasserstoff steht und
$R^4$ für Cyano, Acetyl, Propionyl, Methoxycarbonyl, Ethoxycarbonyl, Dimethylaminocarbonyl, für Methylsulfinyl, Methylsulfonyl, Phenylsulfinyl oder Phenylsulfonyl steht.

Insbesondere bevorzugt sind Verbindungen der Formel (I), bei welchen
R für einen Rest

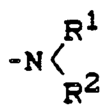

steht,
wobei
$R^1$ für Cyanethyl steht und
$R^2$ für jeweils gegebenenfalls ein- bis dreifach, gleich oder verschieden durch Chlor und Methyl substituiertes Heterocyclylmethyl, Heterocyclylethyl oder Heterocyclylpropyl steht, wobei als Heterocyclylreste infrage kommen:

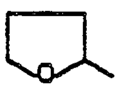 ; 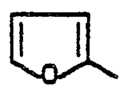 ; 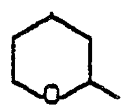 ; 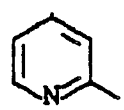 ;

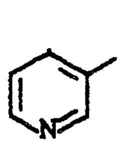 oder 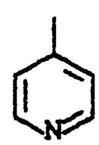 ;

oder für jeweils gegebenenfalls ein- bis dreifach, gleich oder verschieden durch Methyl und Ethyl substituiertes 2-Heterocyclylethyl, 2-Heterocyclylpropyl oder 3-Heterocyclylpropyl steht, wobei als Heterocyclylreste infrage kommen:

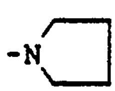 ; 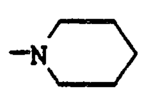 ; 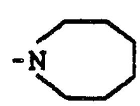 oder 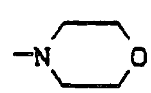 ;

außerdem für Diethylaminoethyl, Dimethylaminopropyl, Diethylaminopropyl, Dibutylaminopropyl oder Diethylaminopentyl, für Cyclohexylmethyl oder Phenethyl steht.

Insbesondere bevorzugt sind daneben auch Verbindungen der Formel (I), bei welchen

R für einen Rest

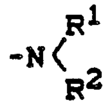

steht,

wobei

$R^1$ für Methyl, Ethyl, n- oder i-Propyl, n-, i-oder s-Butyl, Allyl, Propargyl, für Cyclopropyl, Cyclopentyl, Cyclohexyl, Cyclohexylmethyl, Phenyl, Benzyl oder Cyanoethyl steht und

$R^2$ für gegebenenfalls ein- bis dreifach, gleich oder verschieden durch Chlor und Methyl substituiertes Heterocyclylmethyl steht, wobei als Heterocyclylreste infrage kommen:

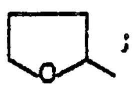 ; 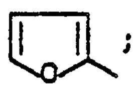 ; 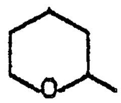 ; 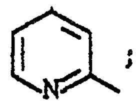 ; 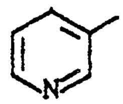

oder 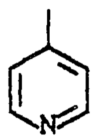 .

Im einzelnen seien außer den bei den Herstellungsbeispielen genannten Verbindungen die folgenden 1-Aminomethyl-3-(2,4-difluor-3-chlorphenyl)-4-cyano-pyrrole der allgemeinen Formel (I) genannt:

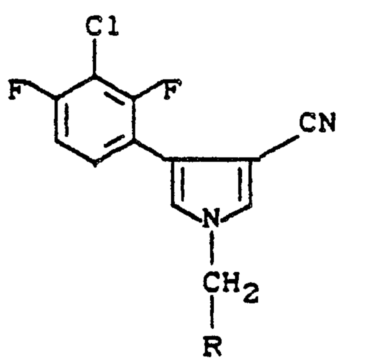

(I)

| R | R |
|---|---|
| 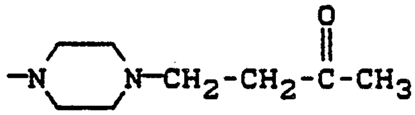 | 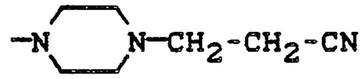 |
| 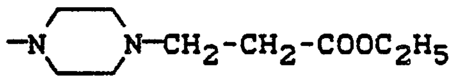 | 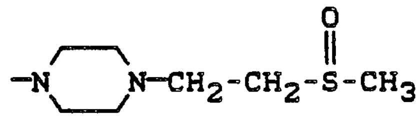 |
| 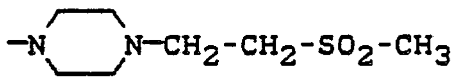 | 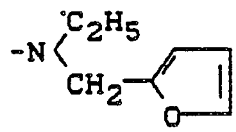 |
| 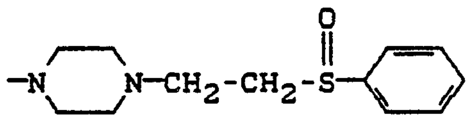 | 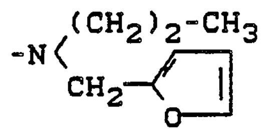 |
| 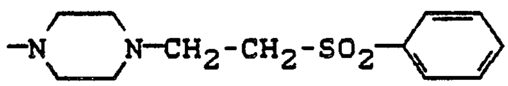 | 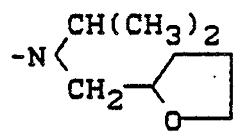 |
| 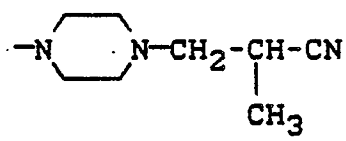 | 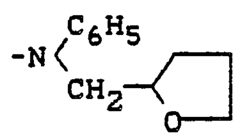 |
| 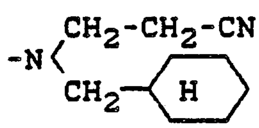 | 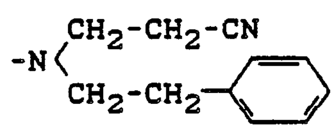 |
| 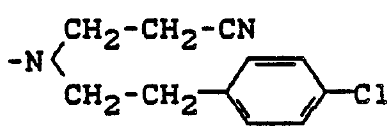 | |

| R | R |
|---|---|
| 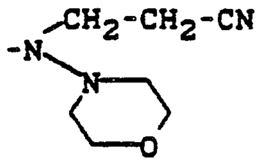 | 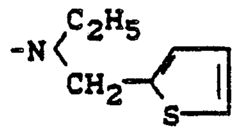 |
| 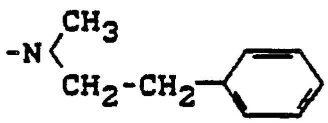 | 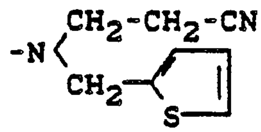 |
| 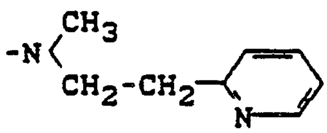 | 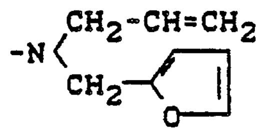 |
| 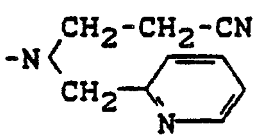 | 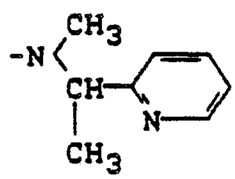 |
| 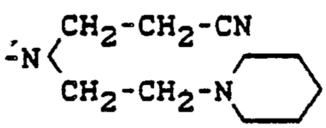 | 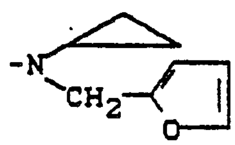 |
| 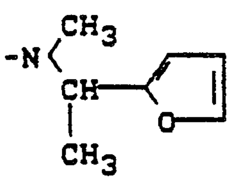 | 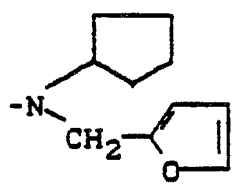 |
| 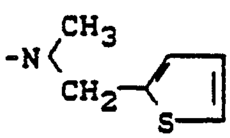 | 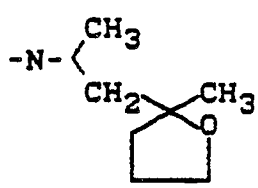 |

| R | R |
|---|---|
| 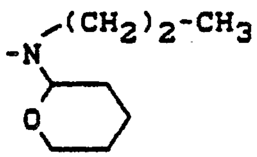 | 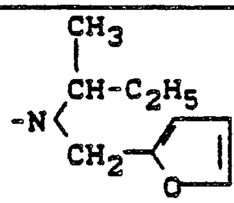 |
| 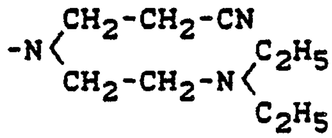 | 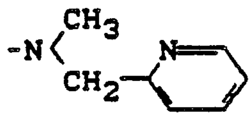 |
| 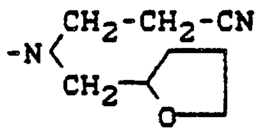 | 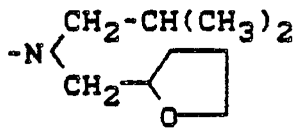 |
| 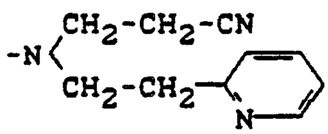 | 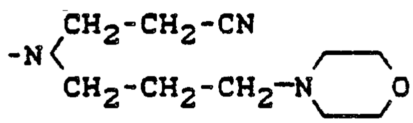 |
| 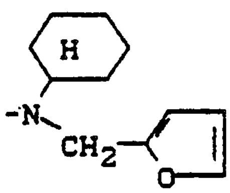 | 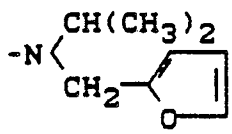 |
| 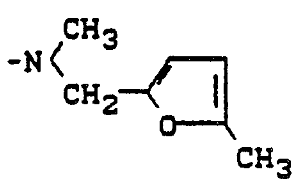 | 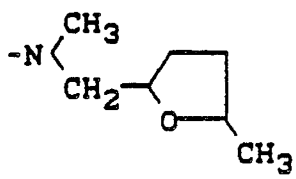 |
| 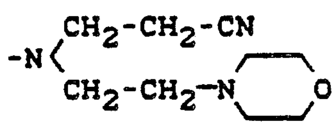 | 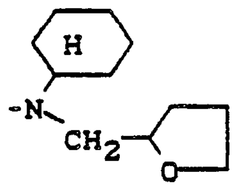 |
| 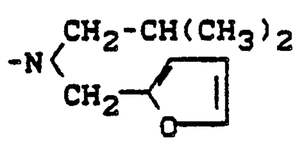 | 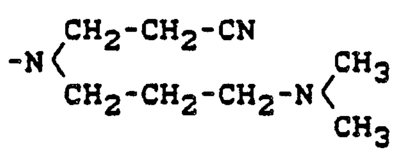 |

| R | R |
|---|---|
| 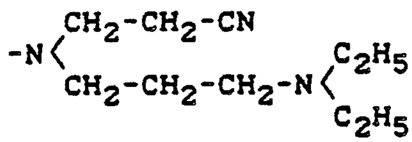 | 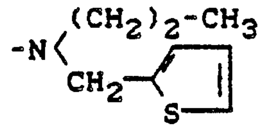 |
| 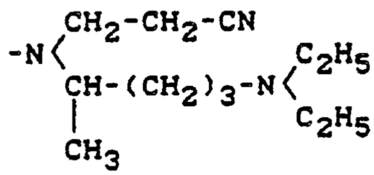 | 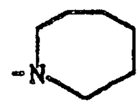 |
| 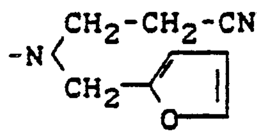 | 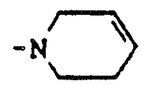 |
| 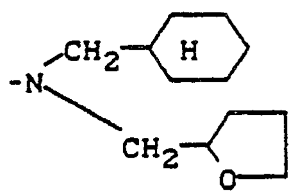 | |
| 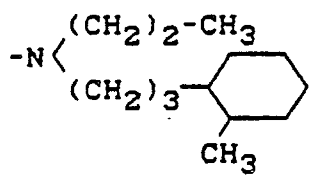 | 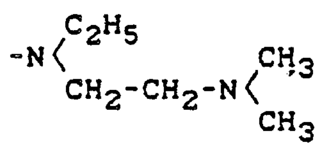 |
| 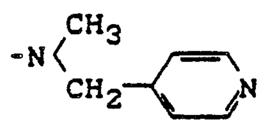 | |
| 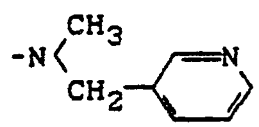 | |
| 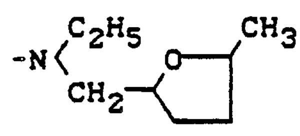 | |

Verwendet man beispielsweise 4-Cyano-3-(2,4-difluor-3-chlorphenyl)-pyrrol, Formaldehyd und Perhydroazepin als Ausgangstoffe, so läßt sich der Reaktionsablauf des erfindungsgemäßen Verfahrens (a) durch das folgende Formelschema darstellen:

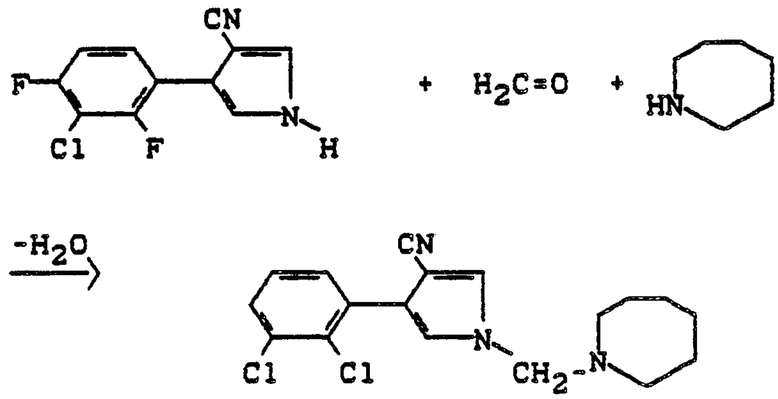


Verwendet man beispielsweise 1-Chlormethyl-4-cyano-3-(2,4-difluor-3-chlorphenyl)-pyrrol und Tetrahydropyridin als Ausgangsverbindungen, so läßt sich der Reaktionsablauf des erfindungsgemäßen Verfahrens (b) durch das folgende Formelschema darstellen:

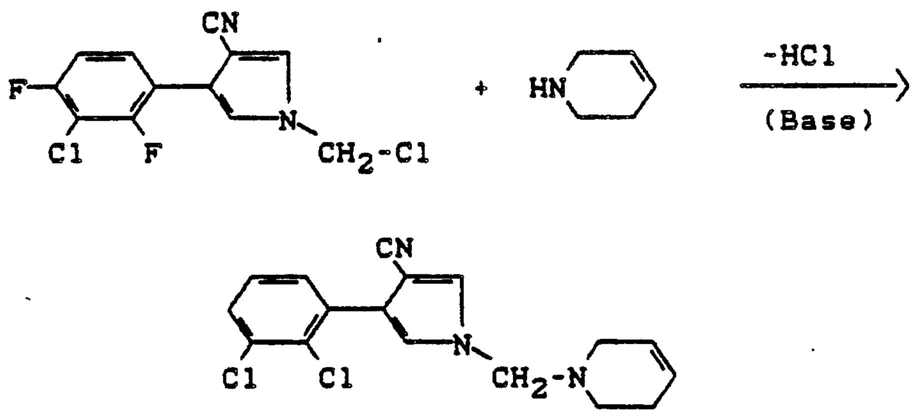


Das zur Durchführung des erfindungsgemäßen Verfahrens (a) als Ausgangsverbindung benötigte 3-(2,4-Difluor-3-chlorphenyl)-4-cyano-pyrrol ist durch die Formel (II) definiert.

Das 3-(2,4-Difluor-3-chlorphenyl)-4-cyano-pyrrol der Formel (II) ist noch nicht bekannt. Es ist jedoch Gegenstand einer eigenen vorgängigen, noch nicht veröffentlichten deutschen Patentanmeldung (P 37 37 983) und erhältlich nach den dort beschriebenen Verfahren, beispielsweise indem man 2,4-Difluor-3-chloranilin der Formel (V)

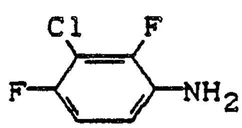
(V)

in allgemeinen üblicher Art und Weise mit Natriumnitrit in Gegenwart von Salzsäure diazotiert, das so erhältliche Diazoniumsalze mit Acrylnitril in Gegenwart eines Kupfer-II-salz-Katalysators umsetzt und das so erhältliche 2-Chlor-3-(2,4-difluor-3-chlorphenyl-propionitril der Formel (VI)

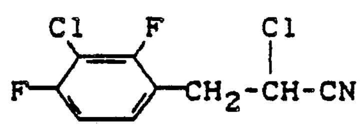
(VI)

mit Basen wie beispielsweise Diazabicycloundecen (DBU) in üblicher Art und Weise dehydrohalogeniert und das so erhältliche Zimtsäurenitril der Formel (VII)

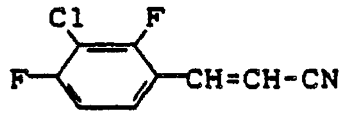

(VII)

mit p-Toluolsulfonylmethylisocyanid der Formel (VIII)

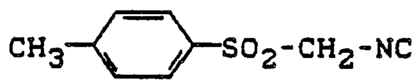

(VIII)

in Gegenwart einer Base wie beispielsweise Natriumhydrid und gegebenenfalls in Gegenwart eines Verdünnungsmittels wie beispielsweise Tetrahydrofuran bei Temperaturen zwischen - 20 °C und + 50 °C umsetzt (vgl. auch die Herstellungsbeispiele).

2,4-Difluor-3-chloranilin der Formel (V) ist bekannt (vgl. z.B. J. Amer. chem. Soc. 81, 94-101 [1959]).

p-Toluolsulfonylmethylisocyanid der Formel (VIII) ist ebenfalls bekannt (vgl. z.B. Synthesis 1985, 400-402; Tetrahedron Lett. 1972, 2367-2368).

Die zur Durchführung des erfindungsgemäßen Verfahrens (b) als Ausgangsstoffe benötigten 3-(2,4-Difluor-3-chlorphenyl)-4-cyano-pyrrole sind durch die Formel (IV) allgemein definiert. In dieser Formel (IV) steht

E vorzugsweise für Hydroxy oder Halogen, insbesondere für Chlor.

Die 3-(2,4-Difluor-3-chlorphenyl)-4-cyano-pyrrole der Formel (IV) sind ebenfalls noch nicht bekannt. Man erhält sie jedoch nach bekannten Verfahren (vgl. EP 133 247), wenn man 3-(2,4-Difluor-3-chlorphenyl)-4-cyano-pyrrol der Formel (II)

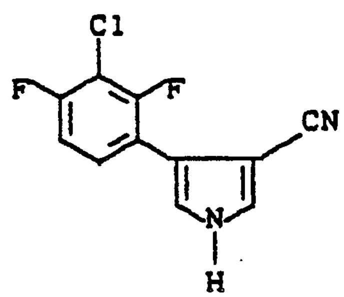

(II)

mit Formaldehyd gegebenenfalls in Gegenwart eines üblichen Verdünnungsmittels wie beispielsweise Tetrahydrofuran und gegebenenfalls in Gegenwart eines Halogenierungsmittels wie beispielsweise Thionylchlorid oder Phosphoroxychlorid sowie gegebenenfalls in Gegenwart eines Säurebindemittels wie beispielsweise Triethylamin oder Diazabicycloundecen (DBU) bei Temperaturen zwischen 20 °C und 160 °C umsetzt.

Die zur Durchführung der erfindungsgemäßen Verfahren (a) und (b) weiterhin als Ausgangsstoffe benötigten Amine sind durch die Formel (III) allgemein definiert. In dieser Formel (III) steht R vorzugsweise für diejenigen Reste, die bereits im Zusammenhang mit der Beschreibung der erfindungsgemäßen Stoffe der Formel (I) als bevorzugt für diesen Substituenten genannt wurden.

Die Amine der Formel (III) sind allgemein bekannte Verbindungen der organischen Chemie oder erhältlich in Analogie zu bekannten Verfahren (vgl. z.B. GB 10 31 916 vom 2.6.1966; Org. Magnet. Res. 7, 488 - 495 [1975]; Kogyo Kagaku Zasshi 63, 1593 - 1597 [1960] bzw. CA 60: 10 542 f).

Als Verdünnungsmittel zur Durchführung des erfindungsgemäßen Verfahrens (a) kommen inerte organische Lösungsmittel oder wässrige Systeme infrage. Vorzugsweise verwendet man protische Lösungsmittel, beispielsweise Alkohole, wie Methanol, Ethanol oder Propanol oder Carbonsäuren, wie Ameisensäure, Essigsäure oder Propionsäure oder deren Gemische mit Wasser. Es ist auch möglich, das erfindungsgemäße Verfahren (a) in aprotischen Lösungsmitteln durchzuführen. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid,

Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (a) wird gegebenenfalls in Gegenwart eines geeigneten Reaktionshilfsmittels durchgeführt. Hierzu eignen sich entweder katalytische bis äquimolare Mengen einer organischen oder anorganischen Säure oder entsprechende Mengen einer geeigneten Base. Als saure Reaktionshilfsmittel kommen insbesondere anorganische Mineralsäuren, wie Phosphorsäure, Schwefel säure, Salpetersäure, Chlorwasserstoffsäure oder Bromwasserstoffsäure oder organische Säuren, wie Ameisensäure, Essigsäure, Propionsäure, Methansulfonsäure, Benzolsulfonsäure oder Toluolsulfonsäure infrage.

Als basische Reaktionshilfsmittel kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Es ist jedoch auch möglich, das als Reaktionspartner verwendete Amin der Formel (III) in entsprechendem Überschuß gleichzeitig als Reaktionshilfsmittel zu verwenden.

Die Reaktionstemperaturen können bei der Durchführung des erfindungsgemäßen Verfahrens (a) in einem größeren Bereich variiert werden. Im allgemeinen arbeitet man bei Temperaturen zwischen 0 °C und 120 °C, vorzugsweise bei Temperaturen zwischen 20 °C und 90 °C.

Zur Durchführung des erfindungsgemäßen Verfahrens (a) setzt man pro Mol an 3-(2,4-Difluor-3-chlorphenyl)-4-cyano-pyrrol der Formel (II) im allgemeinen 1,0 bis 2,0 Mol, vorzugsweise 1,0 bis 1,5 Mol an Amin der Formel (III) und 1,0 bis 2,0 Mol, vorzugsweise 1,0 bis 1,5 Mol an Formaldehyd ein. Der Formaldehyd wird entweder in Form einer wässrigen Lösung, als Paraformaldehyd oder als 1,3,5-Trioxan eingesetzt. Vorzugsweise verwendet man eine wässrige Lösung. Die Reaktionsdurchführung, Aufarbeitung und Isolierung der Reaktionsprodukte der Formel (I) erfolgt in Analogie zu bekannten Verfahren (vgl. EP 133 247).

Als Verdünnungsmittel zur Durchführung des erfindungsgemäßen Verfahrens (b) kommen inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol; Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (b) wird gegebenenfalls in Gegenwart eines geeigneten Säurebindemittels durchge führt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen(DBN) oder Diazabicycloundecen(DBU).

Es ist jedoch auch möglich, das als Reaktionspartner in Frage kommende Amin der Formel (III) in entsprechendem Überschuß gleichzeitig als Reaktionshilfsmittel einzusetzen.

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und 100 °C, vorzugsweise bei Temperaturen zwischen 20 °C und 60 °C.

Zur Durchführung des erfindungsgemäßen Verfahrens (b) setzt man pro Mol an 3-(2,4-Difluor-3-chlorphenyl)-4-cyano-pyrrol der Formel (IV) im allgemeinen 1,0 bis 2,0 Mol, vorzugsweise 1,0 bis 1,5 Mol an Amin der Formel (III) und gegebenenfalls 1,0 bis 2,0 Mol, vorzugsweise 1,0 bis 1,5 Mol an Säurebindemittel ein. Die Reaktionsdurchführung, Aufarbeitung und Isolierung der Reaktionsprodukte der Formel (I) erfolgt in Analogie zu bekannten Verfahren.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind z.B. für den Gebrauch in Pflanzenschutzmitteln, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;

Phytophthora-Arten, wie beispielsweise Phytophthora infestans;

Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Ventirua inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides;
Sclerotinia-Arten, wie z.B. Sclerotinia sclerotiorum.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Reiskrankheiten, wie beispielsweise gegen den Erreger der Reisfleckenkrankheit (Pyricularia oryzae) oder zur Bekämpfung von Krankheiten im Obst- und Gemüsebau, wie beispielsweise gegen den Erreger der Graufäule (Botrytis cinerea) einsetzen. Hervorzuheben ist weiterhin eine gute in vitro-Wirksamkeit der Verbindungen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenak tiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabak stengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

## Herstellungsbeispiele

### Beispiel 1

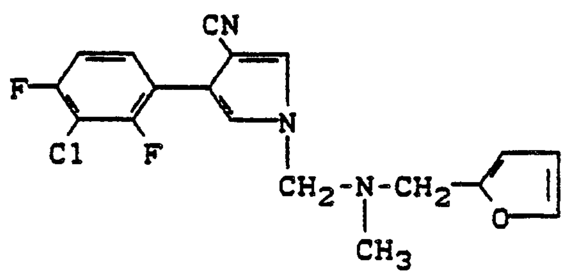

(Verfahren a)

Zu 1,25 g (0,00525 Mol) 4-Cyano-3-(2,4-difluor-3-chlorphenyl)-pyrrol in 10 ml Ethanol gibt man 0,45 g (0,0055 Mol) 37-prozentige wässrige Formaldehydlösung und 0,05 ml Essigsäure. Danach gibt man tropfenweise unter Rühren 0,61 g (0,0055 Mol) N-Methylfurfurylamin zu und rührt nach beendeter Zugabe 20 Stunden bei Raumtemperatur. Zur Aufarbeitung gibt man 50 ml Ethylacetat zu, wäscht dreimal mit Wasser, trocknet über Natriumsulfat, entfernt das Lösungsmittel im Vakuum und kristallisiert den Rückstand aus Ether/Cyclohexan.

Man erhält 1,0 g (53 % der Theorie) an 4-Cyano-3-(2,4-difluor-3-chlorphenyl)-1-(N-methyl-N-furfurylaminomethyl)-pyrrol vom Schmelzpunkt 66 °C - 67 °C.

Herstellung der Ausgangsverbindungen

Beispiel II

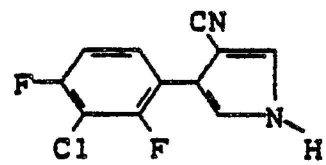

Zu 1,3 g (0.042 Mol) Natriumhydrid (80 %ig in Mineralöl) in 16 ml Tetrahydrofuran unter einer Argonschutzgasatmosphäre gibt man bei - 10 °C bis - 20 °C tropfenweise unter Rühren eine Lösung von 6,0 g (0.0302 Mol) 3-(2,4-Difluor-3-chlorphenyl)-acrylnitril und 7,6 g (0.0392 Mol) p-Toluolsulfonylmethylisocyanid in 20 ml einer Mischung aus Tetrahydrofuran/Dimethylsulfoxid (5:1). Nach beendeter Zugabe läßt man die Reaktionsmischung auf Raumtemperatur kommen, gibt Wasser zu, extrahiert mehrfach mit Essigester, wäscht die vereinigten Essigesterphasen mit Wasser, trocknet über Natriumsulfat und engt im Vakuum ein. Der Rückstand wird durch Chromatographie an Kieselgel (Laufmittel: Cyclohexan/Essigester 5:1) gereinigt.

Man erhält 2,5 g (35 % der Theorie) an 3-Cyano-4-(2,4-Difluor-3-chlorphenyl)-pyrrol vom Schmelzpunkt 218 °C -219 °C.

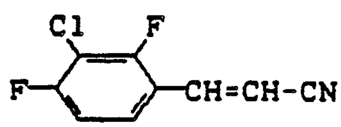

Zu 9,5 g (0.04 Mol) 2-Chlor-3-(2,4-difluor-3-chlorphenyl)-propionitril in 30 ml Tetrahydrofuran tropft man bei Raumtemperatur unter Rühren 6,2 g (0,044 Mol) Diazabicycloundecen in 50 ml Tetrahydrofuran, rührt nach beendeter Zugabe 15 Sunden bei Raumtemperatur, filtriert, engt das Filtrat im Vakuum ein, nimmt den Rückstand in Essigester auf, wäscht nacheinander mit 1 normaler Salzsäure und Wasser, trocknet über Natriumsulfat und entfernt das Lösungsmittel im Vakuum.

Man erhält 7,3 g (91 % der Theorie) an 3-(2,4-Difluor-3-chlorphenyl)-acrylnitril vom Schmelzpunkt 76 °C -77 °C.

Beispiel VI

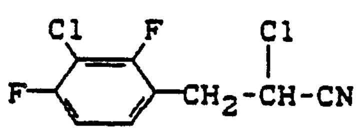

Zu 26,0 g (0.156 Mol) 2,4-Difluor-3-chloranilin in 53 ml Aceton gibt man 53 ml 25prozentige Salzsäure und 37,3 ml (0.479 Mol) Acrylsäurenitril, tropft dann bei 0 °C bis 10 °C unter Rühren innerhalb einer Stunde 11,3 g (0,163 Mol) Natriumnitrit in 21 ml Wasser zu, rührt eine weitere Stunde bei 0 °C bis 10 °C und gibt dann in mehreren Portionen insgesamt 1,3 g (0.016 Mol) Kupfer-II-oxid-Pulver zu, wobei eine heftige Stickstoffgasentwicklung zu beobachten ist. Nach beendeter Gasentwicklung rührt man weitere 15 Stunden bei Raumtemperatur, gibt dan Dichlormethan zu, wäscht mit Wasser, trocknet über natriumsulfat, engt im Vakuum ein und reinigt den öligen Rückstand durch Destillation im Hochvakuum.

Man erhält 6,1 g (25 % der Theorie) an 2-Chlor-3-(2,4-difluor-3-chlorphenyl)-propionitril vom Siedepunkt 75 °C - 77 °C bei 0.15 mbar.

In entsprechender Weise und gemäß den allgemeinen Angaben zur Herstellung erhält man die folgenden 1-Aminomethyl-3-(2,4-difluor-3-chlorphenyl)-4-cyano-pyrrole der allgemeinen Formel (I):

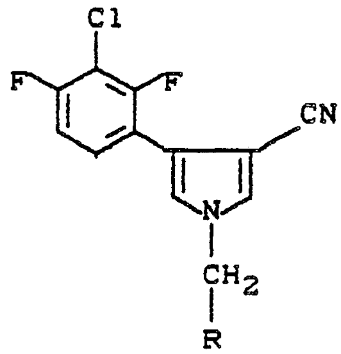

(I)

| Bsp. Nr. | R | physikalische Eigenschaften |
|---|---|---|
| 2 | 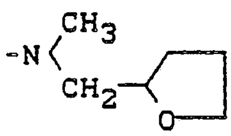 | $^1H$-NMR*): 4,8-5,0 |
| 3 | 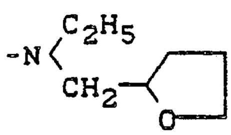 | Fp. 70-71° C |
| 4 | 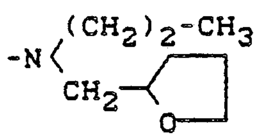 | $^1H$-NMR*): 4,8-5,0 |

| | | |
|---|---|---|
| 5 | 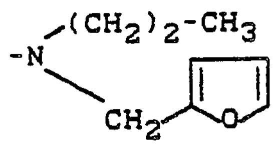 | $^1$H-NMR*): 4,75 |
| 6 | 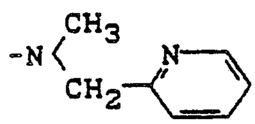 | $^1$H-NMR*): 4,80 |

*) Die $^1$H-NMR-Spektren wurden in $CDCl_3$ mit Tetramethylsilan (TMS) als innerem Standard aufgenommen. Angegeben ist die chemische Verschiebung der Protonen

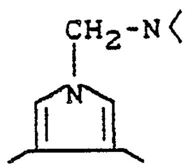 - Gruppe als δ-Wert in ppm.

Beispiel A

Botrytis-Test (Bohne)/protektiv

| | | |
|---|---|---|
| Lösungsmittel: | 4,7 | Gewichtsteile Aceton |
| Emulgator: | 0,3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

In diesem Test zeigt z.B. die Verbindung gemäß Herstellungsbeispielen 1 bis 6 bei 50 ppm Wirkstoffkonzentration einen Wirkungsgrad von 92 % bis 100 %.

Beispiel B

Cochliobolus sativus-Test (Gerste) /protektiv

| | | |
|---|---|---|
| Lösungsmittel: | 100 | Gewichtsteile Dimethylformamid |
| Emulgator: | 0,25 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20° C und 100 % relativer Luftfeuchtigkeit in einer Inkubationskabine.

Die Pflanzen werden in einem Gewächshaus bei einer Temperatur von ca. 20° C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigt bei diesem Test z. B. die Verbindung gemäß Herstellungsbeispiel: 2.

Beispiel C

Pyrenophora teres-Test (Gerste)-protektiv

| | | |
|---|---|---|
| Lösungsmittel: | 100 | Gewichtsteile Dimethylformamid |
| Emulgator: | 0,25 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20° C und 100 % relativer Luftfeuchtigkeit in einer Inkubationskabine.

Die Pflanzen werden in einem Gewächshaus bei einer Temperatur von ca. 20° C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigt bei diesem Test z. B. die Verbindung gemäß Herstellungsbeispiel: 2.

Beispiel D

Leptosphaeria nodorum-Test (Weizen) / protektiv

| | | |
|---|---|---|
| Lösungsmittel: | 100 | Gewichtsteile Dimethylformamid |
| Emulgator: | 0,25 | Gewichtsteile Alkyarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensuspension von Leptosphaeria nodorum besprüht. Die Pflanzen verbleiben 48 Stunden bei 20° C und 100 % relativer

Luftfeuchtigkeit in einer Inkubationskabine.

Die Pflanzen werden in einem Gewächshaus bei einer Temperatur von ca. 15° C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigt bei diesem Test z. B. die Verbindung gemäß Herstellungsbeispiel: 2.

## Ansprüche

1. 1-Aminomethyl-3-(2,4-difluor-3-chlorphenyl)-4-cyano-pyrrole der allgemeinen Formel (I),

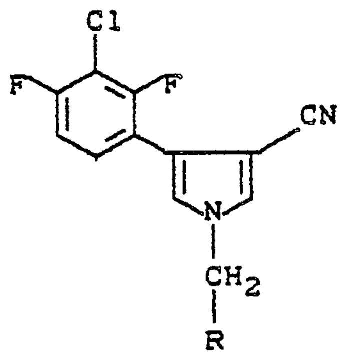

(I)

in welcher
R für einen Rest

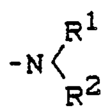 ; 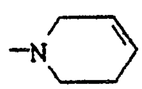 ; 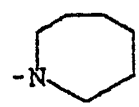

oder

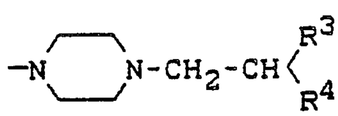

steht,
wobei
$R^1$ für gegebenenfalls substituiertes Alkyl, für Alkenyl, Alkinyl oder Cycloalkyl oder für jeweils gegebenenfalls substituiertes Aralkyl oder Aryl steht,
$R^2$ für jeweils gegebenenfalls substituiertes Heterocyclylalkyl, Heterocyclylalkenyl, Heterocyclylalkinyl oder Heterocyclyl, für Dialkylaminoalkyl oder für jeweils gegebenenfalls substituiertes Cycloalkylalkyl oder Phenethyl steht,
$R^3$ für Wasserstoff oder Alkyl steht und
$R^4$ für Cyano, Alkanoyl, Alkoxycarbonyl, Alkylaminocarbonyl, Dialkylaminocarbonyl, für Alkylsulfinyl, für Alkylsulfonyl, für Phenylsulfinyl oder für Phenylsulfonyl steht.

2. 1-Aminomethyl-3-(2,4-difluor-3-chlorphenyl)-4-cyano-pyrrole gemäß Anspruch 1, wobei in der Formel (I)
R für einen Rest

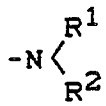 ; 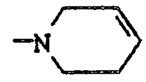 ; 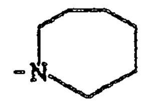

oder

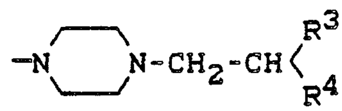

steht,

wobei

$R^1$ für gegebenenfalls substituiertes, geradkettiges oder verzweigtes Alkyl mit 1 bis 6 Kohlenstoffatomen steht, wobei als Substituenten infrage kommen: Cyano, Cycloalkyl mit 3 bis 7 Kohlenstoffatomen, jeweils geradkettiges oder verzweigtes Alkoxy, Alkylcarbonyl, Alkoxycarbonyl, Alkylaminocarbonyl, Dialkylaminocarbonyl, Alkylsulfinyl, Alkylsulfonyl oder Dialkylamino mit jeweils 1 bis 6 Kohlenstoffatomen in den einzelnen Alkylteilen; außerdem für jeweils geradkettiges oder verzweigtes Alkenyl oder Alkinyl mit jeweils 3 bis 6 Kohlenstoffatomen, für Cycloalkyl mit 3 bis 7 Kohlenstoffatomen oder für jeweils gegebenenfalls einfach bis mehrfach, gleich oder verschieden im Phenylteil substituiertes Phenyl oder Benzyl steht, wobei als Substituenten jeweils infrage kommen: Halogen, Cyano, jeweils geradkettiges oder verzweigtes Alkyl, Alkoxy, Halogenalkyl, Halogenalkoxy, Alkylcarbonyl oder Alkoxycarbonyl mit jeweils 1 bis 6 Kohlenstoffatomen in den einzelnen Alkylteilen und im Fall des Halogenalkyl- oder Halogenalkoxy-Restes mit jeweils 1 bis 9 gleichen oder verschiedenen Halogenatomen,

$R^2$ für jeweils geradkettiges oder verzweigtes Heterocyclylalkyl mit 1 bis 6 Kohlenstoffatomen im Alkylteil, Heterocyclylalkenyl mit 3 bis 6 Kohlenstoffatomen im Alkenylteil, Heterocyclylalkinyl mit 3 bis 6 Kohlenstoffatomen im Alkinylteil oder Heterocyclyl steht, wobei Heterocyclyl jeweils für einen 5-bis 7-gliedrigen, gegebenenfalls einfach bis mehrfach, gleich oder verschieden substituierten, ungesättigten oder gesättigten Heterocyclus mit 1 bis 3 Heteroatomen, insbesondere Stickstoff, Sauerstoff oder Schwefel steht und wobei als Substituenten jeweils infrage kommen: Halogen, Cyano, jeweils geradkettiges oder verzweigtes Alkyl, Alkoxy, Halogenalkyl, Halogenalkoxy, Alkylcarbonyl oder Alkoxycarbonyl mit jeweils 1 bis 6 Kohlenstoffatomen in den einzelnen Alkylteilen und im Fall des Halogenalkyl- oder Halogenalkoxy-Restes mit jeweils 1 bis 9 gleichen oder verschiedenen Halogenatomen; außerdem für geradkettiges oder verzweigtes Dialkylaminoalkyl mit jeweils 1 bis 6 Kohlenstoffatomen in den einzelnen Alkylteilen steht, ferner für im Cycloalkylteil gegebenenfalls einfach bis mehrfach, gleich oder verschieden durch Halogen und/oder geradkettiges oder verzweigtes Alkyl mit 1 bis 4 Kohlenstoffatomen substituiertes Cycloalkylalkyl mit 1 bis 6 Kohlenstoffatomen im geradkettigen oder verzweigten Alkylteil und 3 bis 7 Kohlenstoffatomen im Cycloalkylteil oder für im Phenylteil gegebenenfalls einfach bis mehrfach, gleich oder verschieden durch Cyano, Halogen und/oder jeweils geradkettiges oder verzweigtes Alkyl oder Alkoxy mit jeweils 1 bis 4 Kohlenstoffatomen substituiertes Phenylethyl steht,

$R^3$ für Wasserstoff oder für geradkettiges oder verzweigtes Alkyl mit 1 bis 6 Kohlenstoffatomen steht und

$R^4$ für Cyano oder für jeweils geradkettiges oder verzweigtes Alkanoyl, Alkoxycarbonyl, Alkylaminocarbonyl, Dialkylaminocarbonyl, Alkylsulfinyl oder Alkylsulfonyl mit jeweils 1 bis 6 Kohlenstoffatomen in den einzelnen Alkylteilen oder für Phenylsulfinyl oder Phenylsulfonyl steht.

3. 3-Aminomethyl-3-(2,4-difluor-3-chlorphenyl)-4-cyano-pyrrole gemäß Anspruch 1, wobei in der Formel (I)

R für einen Rest

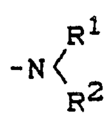 ; 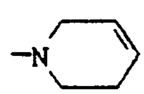 ; 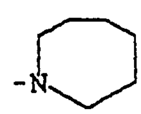

oder

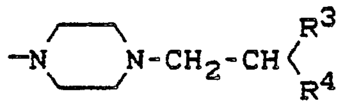

steht,
wobei
$R^1$ für jeweils gegebenenfalls substituiertes Methyl, Ethyl, n- oder i-Propyl, n-, i-oder s-Butyl steht, wobei als Substituenten jeweils infrage kommen: Cyano, Methoxy, Ethoxy, methylcarbonyl, Ethylcar bonyl, Methoxycarbonyl, Ethoxycarbonyl, Methylaminocarbonyl, Dimethylaminocarbonyl, Ethylaminocarbonyl, Diethylaminocarbonyl, Methylsulfinyl, Methylsulfonyl, Dimethylamino, Diethyl-amino oder Dipropylamino, Dibutylamino oder Cyclohexyl; außerdem für Allyl, n- oder i-Butenyl, Propargyl, n- oder i-Butinyl, für Cyclopentyl, Cyclohexyl, Cyclopropyl oder für jeweils gegebenenfalls ein- bis dreifach, gleich oder verschieden im Phenylteil substituiertes Benzyl oder Phenyl steht, wobei als Substituenten infrage kommen: Fluor, Chlor, Brom, Cyano, Methyl, Ethyl, n-oder i-Propyl, n-, i- oder s-Butyl, Methoxy, Ethoxy, Trifluormethyl, Trifluormethoxy, Acetyl, Methoxycarbonyl oder Ethoxycarbonyl,
$R^2$ für jeweils gegebenenfalls im Heterocyclylteil ein- oder zweifach, gleich oder verschieden substituiertes Heterocyclylmethyl, Heterocyclylethyl oder Heterocyclylpropyl steht, wobei als Heterocyclylrest jeweils einer der folgenden Rest infrage kommt

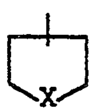 ; 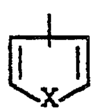 ; 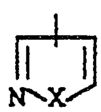 ; 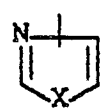 ; 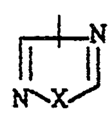 ;

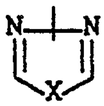 ; 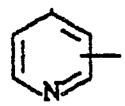 ; 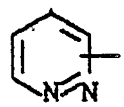 ; 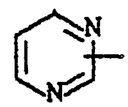 ; 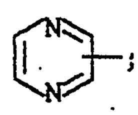 ;

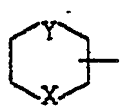 und 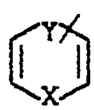 ,

wobei X jeweils für Sauerstoff, Schwefel oder eine NH-Gruppe steht und Y jeweils für Sauerstoff, Schwefel, eine NH-Gruppe oder eine $CH_2$-Gruppe steht und wobei als Substituenten jeweils infrage kommen: Fluor, Chlor, Brom, Cyano, Methyl, Ethyl, n- oder i-Propyl, n-, i-, s- oder t-Butyl, Methoxy, Ethoxy, Trifluormethyl, Trifluormethoxy, Acetyl, Methoxycarbonyl oder Ethoxycarbonyl; außerdem für Dimethylaminoethyl, Diethylaminoethyl, Dimethylaminopropyl, Diethylaminopropyl, Dipropylaminopropyl, Dibutylaminopropyl, Dibutylaminobutyl, Dibutylaminoethyl, Dipropylaminoethyl, Dimethylaminobutyl, Diethylaminobutyl, Dimethylaminopentyl, Diethylaminopentyl, für jeweils gegebenenfalls ein- bis dreifach, gleich oder verschieden durch Methyl, Ethyl und/oder Isopropyl substituiertes Cyclohexylmethyl oder Cyclohexylethyl oder für gegenbenenfalls ein- bis dreifach, gleich oder verschieden durch Fluor, Chlor, Brom, Cyano, Methyl, Ethyl, n- oder i-Propyl, n-, i-, s- oder t-Butyl, Methoxy, Ethoxy, n- oder i-Propoxy und n-, i-, s- oder t-Butoxy substituiertes Phenethyl steht,
$R^3$ für Wasserstoff, Methyl, Ethyl, n- oder i-Propyl, n-, i-, s- oder t-Butyl steht und
$R^4$ für Cyano, Acetyl, Propionyl, Methoxycarbonyl, Ethoxycarbonyl, Methylaminocarbonyl, Dimethylaminocarbonyl, Ethylaminocarbonyl, Diethylaminocarbonyl, Dipropylaminocarbonyl, Dibutylaminocarbonyl, Methylsulfinyl, Ethylsulfinyl, Methylsulfonyl, Ethylsulfonyl, Phenylsulfinyl oder Phenylsulfonyl steht.

4. 1-Aminomethyl-3-(2,4-difluor-3-chorphenyl)-4-cyano-pyrrole gemäß Anspruch 1, wobei in der Formel (I)
R für einen Rest

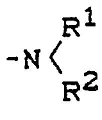 ; 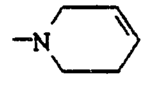 ; 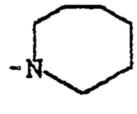

oder

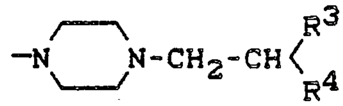

steht,
wobei
$R^1$ für Methyl, Ethyl, n- oder i-Propyl, n-, i- oder s-Butyl, Allyl, Propargyl, für Cyclohexyl, Cyclopropyl, Cyclopentyl, Cyclohexylmethyl, Phenyl, Benzyl oder für Cyanethyl steht,
$R^2$ für jeweils gegebenenfalls ein- bis dreifach, gleich oder verschieden durch Chlor und Methyl substituiertes Heterocyclylmethyl, Heterocyclylethyl oder Heterocyclylpropyl steht, wobei als Heterocyclylreste infrage kommen:

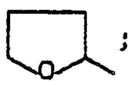 ; 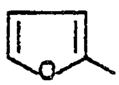 ; 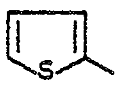 ; 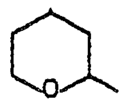 ; 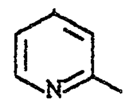 ;

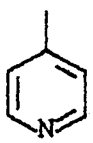 oder 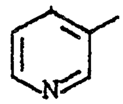 ;

oder für jeweils gegebenenfalls ein- bis dreifach, gleich oder verschieden durch Methyl und Ethyl substituiertes 2-Heterocyclylethyl, 2-Heterocyclylpropyl oder 3-Heterocyclylpropyl steht, wobei als Heterocyclylreste infrage kommen:

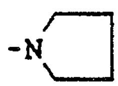 ; 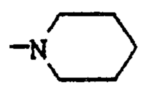 ; 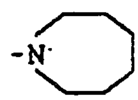 ; 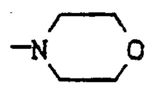 ;

außerdem für Diethylaminoethyl, Dimethylaminopropyl, Diethylaminopropyl, Dibutylaminopropyl oder Diethylaminopentyl, für Cyclohexylmethyl oder für Phenethyl steht,
$R^3$ für Wasserstoff steht und
$R^4$ für Cyano, Acetyl, Propionyl, Methoxycarbonyl, Ethoxycarbonyl, Dimethylaminocarbonyl, für Methylsulfinyl, Methylsulfonyl, Phenylsulfinyl oder Phenylsulfonyl steht.

5. 1-Aminomethyl-3-(2,4-difluor-3-chlorphenyl)-4-cyano-pyrrole gemäß Anspruch 1, wobei in der Formel (I),
R für einen Rest

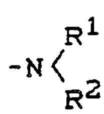

steht,
wobei
$R^1$ für Cyanethyl steht und
$R^2$ für jeweils gegebenenfalls ein- bis dreifach, gleich oder verschieden durch Chlor und Methyl substituiertes Heterocyclylmethyl, Heterocyclylethyl oder Heterocyclylpropyl steht, wobei als Heterocyclylreste infrage kommen:

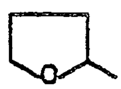 ; 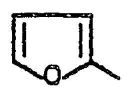 ; 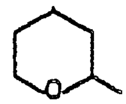 ; 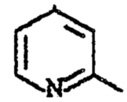 ;

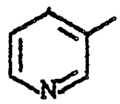 oder 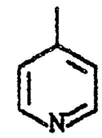 ;

oder für jeweils gegebenenfalls ein- bis dreifach, gleich oder verschieden durch Methyl und Ethyl substituiertes 2-Heterocyclylethyl, 2-Heterocyclylpropyl oder 3-Heterocyclylpropyl steht, wobei als Heterocyclylreste infrage kommen:

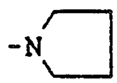 ; 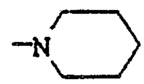 ; 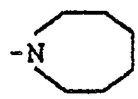 oder 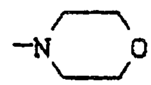 ;

außerdem für Diethylaminoethyl, Dimethylaminopropyl, Diethylaminopropyl, Dibutylaminopropyl oder Diethylaminopentyl, für Cyclohexylmethyl oder Phenethyl steht.

6. 1-Aminomethyl-3-(2,4-difluor-3-chlorphenyl)-4-cyano-pyrrole gemäß Anspruch 1, wobei in der Formel (I)
R für einen Rest

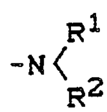

steht,
wobei
$R^1$ für Methyl, Ethyl, n- oder i-Propyl, n-, i- oder s-Butyl, Allyl, Propargyl, für Cyclopropyl, Cyclopentyl, Cyclohexyl, Cyclohexylmethyl, Phenyl, Benzyl oder Cyanoethyl steht und
$R^2$ für gegebenenfalls ein- bis dreifach, gleich oder verschieden durch Chlor und Methyl substituiertes Heterocyclylmethyl steht, wobei als Heterooyclylreste infrage kommen:

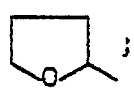 ; 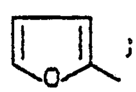 ; 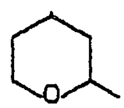 ; 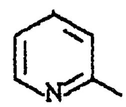 ; 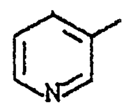

oder 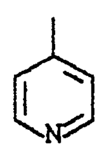 .

7. Verfahren zur Herstellung von 1-Aminomethyl-3-(2,4-difluor-3-chlorphenyl)-4-cyano-pyrrolen der allgemeinen Formel (I),

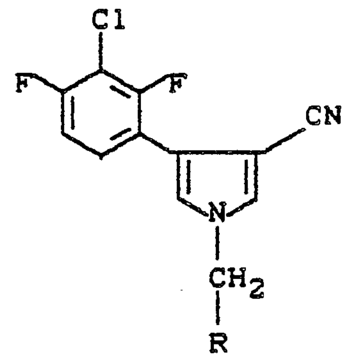

(I)

in welcher
R für einen Rest

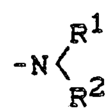 ; 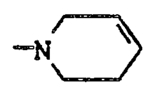 ; 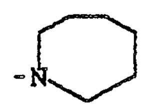

oder

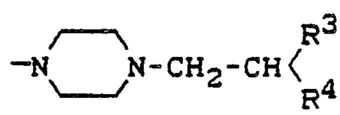

steht,
wobei
$R^1$ für gegenbenenfalls substituiertes Alkyl, für Alkenyl, Alkinyl oder Cycloalkyl oder für jeweils gegebenenfalls substituiertes Aralkyl oder Aryl steht,
$R^2$ für jeweils gegebenenfalls substituiertes Heterocyclylalkyl, Heterocyclylalkenyl, Heterocyclylalkinyl oder Heterocyclyl, für Dialkylaminoalkyl oder für jeweils gegebenenfalls substituiertes Cycloalkylalkyl oder Phenethyl steht,
$R^3$ für Wasserstoff oder Alkyl steht und
$R^4$ für Cyano, Alkanoyl, Alkoxycarbonyl, Alkylaminocarbonyl, Dialkylaminocarbonyl, für Alkylsulfinyl, für Alkylsulfonyl, für Phenylsulfinyl oder für Phenylsulfonyl steht, dadurch gekennzeichnet, daß man
(a) das 3-(2,4-Difluor-3-chlorphenyl)-4-cyano-pyrrol der Formel (II)

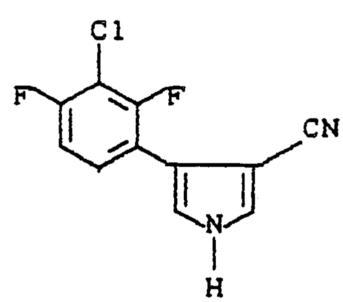

(II)

mit Formaldehyd und Aminen der Formel (III),
R-H (III)
in welcher
R die oben angegebene Bedeutung hat,
gegebenenfalls in Gegenwart eines Verdünnungsmittels und gegebenenfalls in Gegenwart eines Reaktionshilfsmittels umsetzt oder daß man

b) 3-(2,4-Difluor-3-chlorphenyl)-4-cyano-pyrrole der Formel (IV),

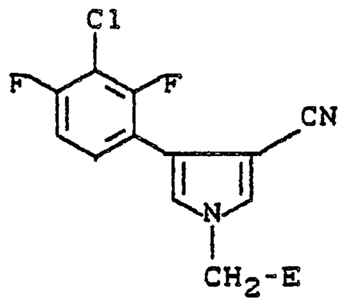

(IV)

in welcher

E für eine elektronenanziehende Abgangsgruppe steht,

mit Aminen der Formel (III),

H-R (III)

in welcher

R die oben angegebene Bedeutung hat,

gegebenenfalls in Gegenwart eines Verdünnungsmittels und gegebenenfalls in Gegenwart eines Säurebindemittels umsetzt.

8. Schädlingsbekämpfungsmittel, gekennzeichnet durch einen Gehalt an mindestens einem 1-Aminomethyl-3-(2,4-difluor-3-chlorphenyl)-4-cyano-pyrrol der Formel (I) nach den Ansprüchen 1 und 7.

9. Verwendung von 1-Aminomethyl-3-(2,4-difluor-3-chlorphenyl)-4-cyano-pyrrolen der Formel (I) nach den Ansprüchen 1 und 7 zur Bekämpfung von Schädlingen.

10. Verfahren zur Bekämpfung von Schädlingen, dadurch gekennzeichnet, daß man 1-Aminomethyl-(2,4-difluor-3-chlorphenyl)-4-cyano-pyrrole der Formel (I) nach den Ansprüchen 1 und 7 auf Schädlinge und/oder ihren Lebensraum einwirken läßt.

11. Verfahren zur Herstellung von Schädlingsbekämpfungsmitteln, dadurch gekennzeichnet, daß man 1-Aminomethyl-3-(2,4-difluor-3-chlorphenyl)-4-cyanopyrrole der Formel (I) nach den Ansprüchen 1 und 7 mit Streckmitteln und/oder oberflächenaktiven Mitteln vermischt.

12. 3-(2,4-Difluor-3-chlorphenyl)-4-cyano-pyrrole der Formel (IV),

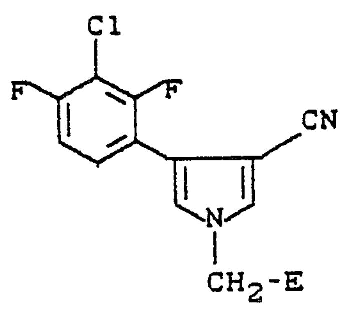

(IV)

in welcher

E für eine elektronenanziehende Abgangsgruppe steht.

13. Verfahren zur Herstellung von 3-(2,4-Difluor-3-chlorphenyl)-4-cyano-pyrrolen der Formel (IV),

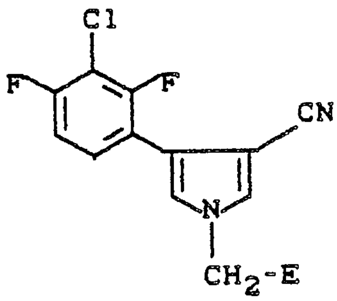

(IV)

in welcher
E für eine elektronenanziehende Abgangsgruppe steht,
dadurch gekennzeichnet, daß man 3-(2,4-Difluor-3-chlorphenyl)-4-cyano-pyrrol der Formel (II)

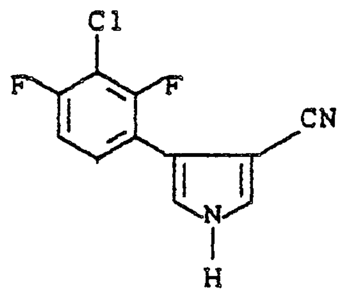

(II)

mit Formaldehyd gegebenenfalls in Gegenwart eines Verdünnungsmittels und gegebenenfalls in Gegenwart eines Halogenierungsmittels, sowie gegebenenfalls in Gegenwart eines Säurebindemittels bei Temperaturen zwischen 20° C und 160° C umsetzt.

Europäisches Patentamt

# EUROPÄISCHER RECHERCHENBERICHT

Nummer der Anmeldung

EP 89 10 1842

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y,D | EP-A-0 133 247 (CIBA-GEIGY)<br>* Ansprüche 1,11,12 *<br>--- | 1,9 | C 07 D 401/12<br>C 07 D 405/12<br>C 07 D 409/12<br>C 07 D 207/34<br>A 01 N 43/36<br>A 01 N 43/40 |
| Y | EP-A-0 145 095 (NIPPON SODA)<br>* Ansprüche 1,10 *<br>----- | 1,9 | |

| RECHERCHIERTE SACHGEBIETE (Int. Cl.4) |
|---|
| C 07 D 401/00<br>C 07 D 405/00<br>C 07 D 409/00<br>C 07 D 207/00<br>A 01 N 43/00 |

Der vorliegende Recherchenbericht wurde für alle Patentansprüche erstellt

| Recherchenort | Abschlußdatum der Recherche | Prüfer |
|---|---|---|
| DEN HAAG | 12-05-1989 | CASADO Y MARTIN DE MERCA |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)